(19) Europäisches Patentamt
European Patent Office
Office européen des brevets

(11) Publication number: **0 316 018 A2**

# (12) EUROPEAN PATENT APPLICATION

(21) Application number: 88120140.4

(51) Int. Cl.4: **C12N 15/00**

(22) Date of filing: 20.02.86

(30) Priority: 29.03.85 US 717319

(43) Date of publication of application:
17.05.89 Bulletin 89/20

(60) Publication number of the earlier application in accordance with Art.76 EPC: 0 196 762

(84) Designated Contracting States:
AT BE CH DE FR GB IT LI NL SE

(71) Applicant: CETUS CORPORATION
1400 Fifty-Third Street
Emeryville California 94608(US)

(72) Inventor: Horn, Glenn Thomas
3 Admiral Drive No. F370
Emeryville California 94608(US)
Inventor: Piatak, Michael, Jr.
1120 Alfred Avenue
Walnut Creek California 94596(US)

(74) Representative: Bizley, Richard Edward et al
BOULT, WADE & TENNANT 27 Furnival Street
London EC4A 1PQ(GB)

## (54) Modification of DNA sequences.

(57) The invention is embodied by digesting a vector DNA sequence with a restriction enzyme, removing any unwanted nucleotides, treating the cleaved vector with exonuclease III to create 5′ sticky ends, mixing with the thus treated vector an even number of single-stranded nucleotides of alternating sense and anti-sense sequence which alternately encode or are complementary to the desired sequences in the product DNA, and which single-stranded ologinucleotides have termini which overlap and are complementary to termini of the adjacent oligonucleotide(s) and/or cleaved vector 5′ sticky ends so as to form a tendem bridge between the 5′ sticky end upstream of the original cleavage site and the 5′ sticky end downstream of the original cleavage site, and treating the mixture with DNA polyemrase and ligase.

EP 0 316 018 A2

# THE MODIFICATION OF DNA SEQUENCES

This invention relates to a method of modifying DNA sequences.

Although broadly applicable, the techniques and materials of the present invention permit, inter alia and by way of example, selective modification of the amino acid sequence of the B chain ricin and thus permit manipulation to provide properties which are capable of enhancing the cytoxicity of ricin or of other toxins and the derivatives thereof.

Copending European Patent Application No. 86301227.4, from which this application is divided, describes and claims ricin prepared using recombinant techniques. The present specification incorporates the disclosure of the parent case by reference. The amino acid sequence of the ricin B can be, if desired, absolutely identical to the ricin B peptide amino acid sequence as extracted from castor bean seeds, but the recombinant product is inevitably somewhat modified due to the environment of its production, and may be further modified at the will of the producer to contain alternations in amino acid sequence using the present invention or in the level of glycosylation.

Said copending Application describes the preparation of the coding sequence for the ricin B protein using a novel sequence of reactions. A cloning vector containing a substantial portion of the ricin B encoding sequence was modified by digestion at a restriction site proximal to the 5′ end of the cDNA insert in the cloning vector, followed by, as was necessary in this case, digestion with S1 nuclease sufficient to remove nucleotides intervening before the start of the cDNA insert, and treating with exonuclease III to provide 5′ sticky ends. The sticky-ended vector was then treated with a mixture of two oligonucleotides, each of which contained a portion of the sequence required to complete the coding sequence for the N-terminus of the desired ricin B protein, and each of which had sufficient regions of overlap with respect to each other, and with respect to the 5′ sticky ends of the cDNA sequence and of the cleaved vector sequence to form a tandem bridge of mostly single-stranded sequences. When made double-stranded by the action of DNA polymerase I large fragment (Klenow) and treated with ligase, these oligonucleotides and the exonuclease III treated vector generated the missing portions of the cDNA insert so as to provide the entire coding sequence for the desired protein, along with a convenient restriction site 5′ of the start codon, and religated the vector.

This procedure offers a broadly applicable method to complete a desired DNA sequence or in general to modify, delete or add sequence to a double-stranded DNA to give a desired product DNA. Thus, the method of the present invention comprises digesting a vector DNA sequence with a restriction enzyme, removing any unwanted nucleotides, treating the cleaved vector with exonuclease III to create 5′ sticky ends, mixing with the thus treated vector an even number of single-stranded nucleotides of alternating sense and anti-sense sequence which alternately encode or are complementary to the desired sequences in the product DNA, and which single-stranded ologinucleotides have termini which overlap and are complementary to termini of the adjacent oligonucleotide(s) and/or cleaved vector 5′ sticky ends so as to form a tandem bridge between the 5′ sticky end upstream of the original cleavage site and the 5′ sticky end downstream of the original cleavage site, and treating the mixture with DNA polymerase I (Klenow fragment) and ligase. In the following description, the method of the invention will be described in relation to work on ricin B and the preparation of a cassette encoding ricin B.

## Brief Description of the Drawings

Figure 1 is a diagramatic representation process for completing the coding sequence of an isolated cDNA using tandem single-stranded oligonucleotide bridges.

Figure 2 shows the protein sequence of ricin B as disclosed by Funatsu (supra) and obtained from the extracted protein.

Figure 3 shows the nucleotide sequence of the cDNA insert of the plasmid pRTB5 corresponding to the ricin B chain partial coding sequence, along with the amino acid sequence deduced from it. Also shown for comparison is the sequence of ricin B as determined from the extracted protein by Funatsu.

Figure 4a & 4b shows a comparison of base (4a) and protein (4b) sequence of the aforementioned cDNA insert with a cDNA insert of the plasmid pRTB4 which encodes a major portion of the sequence of the B portion of RCA. Also shown is the sequence of pRTA115, which overlaps a portion of the pRTB5 sequence.

Figure 5 shows the sequences of the synthetic oligonucleotides used to complete the coding sequence of ricin B derived from pRTB5.

Figure 6 shows diagrammatically the construction of pRTB601.

## Definitions

"Operably linked" when used in describing DNA sequences refers to juxtaposition in such a way that the functionality of the sequences is preserved. Thus, for example a coding sequence "operably linked" to a promoter is positioned so that the promoter is capable of effecting the expression of the coding sequence.

"Control" sequence refers to those DNA sequences which control initiation and termination of transcription and translation. In procaryotic systems, for example, control sequences comprise promoter or promoter/operator and nucleotides encoding a ribosome binding site.

"Recombinant host cells" refers to cells which have been transformed with DNA sequences constructed by recombinant techniques. Such reference includes both the cells as separated, for example by filtration or as a centrifugation pellet, and to cultures of these cells. Indeed, "cells" and "cell cultures," where the context so permits, are used interchangeably herein.

"Upstream cleavage terminus" and "downstream cleavage terminus" refer to the DNA sequence ends resulting when a double-stranded DNA is cleaved with a restriction enzyme. Depending on the enzyme used, the ends may be sticky or blunt. Using the usual convention, for the "upstream" terminus, the sense strand terminates with 3′, and the anti-sense strand terminates with 5′; the opposite is true for the "downstream terminus".

"Comprised alternately of sense and anti-sense sequences" when used to describe a series of single-stranded oligonucleotides means that when arranged "approximately" end-to-end the oligonucleotides taken together will provide an entire sequence with each oligonucleotide sequence picking up in turn with sense-anti-sense/sense/anti-sense, etc., ie,

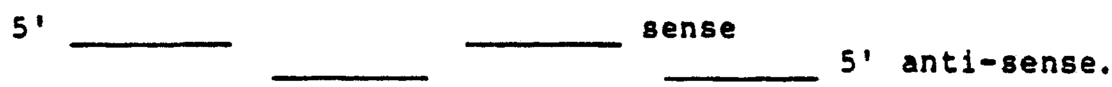


By "approximately" in the foregoing paragraph is meant that the oligonucleotides may further overlap for no more than 89% of their length so that hybridization can occur at the termini, ie,

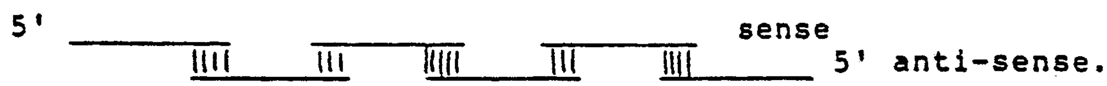


"Contiguous with" when used to describe the relation of DNA sequences refers to a relationship such that one sequence is a continuation of the other, or complementary to a continuation of the other. However, the continuity may be "approximate" in the sense of the previous paragraph.

The approach followed to obtain a cassette encoding ricin B is, briefly, as follows:

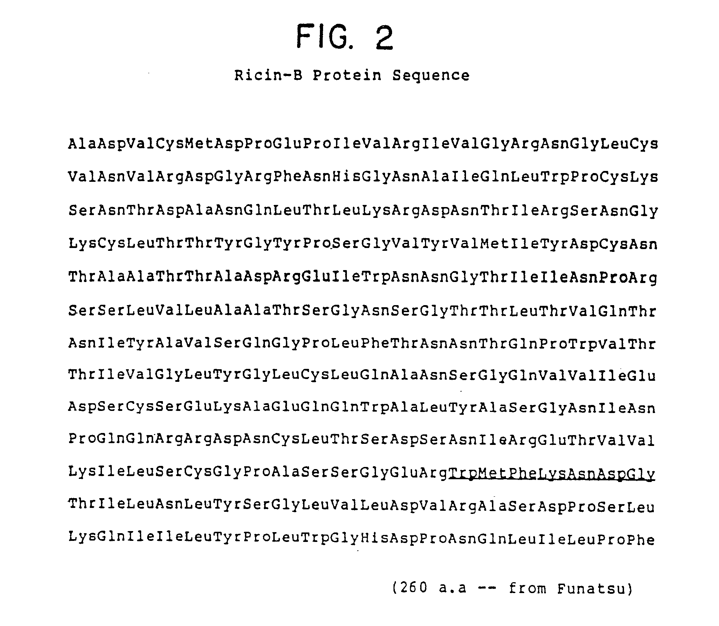

1. The sequence published for naturally-occurring ricin B showed the presence of the amino acid sequence, Trp-Met-Phe-Lys-Asn-Asp-Gly (see Fig. 2), which is associated with minimal codon redundancy. A mixture of all oligonucleotide sequences encoding this sequence was constructed as a probe.

2. A cDNA library was constructed by isolating mRNA from castor bean seeds, and preparing the corresponding cDNA by, in general, conventional methods. However, during the construction, appropriate linkers were ligated to the ends of the cDNA so as to obtain inserts bounded by EcoRI/SalI sites. EcoRI/SalI inserts were then ligated into the cloning vector, pUC13, and transformed into E. coli. Successful transformants capable of hybridizing with the probe were selected and sequenced.

3. Colonies were obtained which contained large portions of the ricin B and agglutinin B sequences. In addition, a colony was obtained which contained the sequences for a portion of the putative peptide precursor of both RCA and ricin which was thus shown to contain a twelve amino acid bridging peptide. The cDNA insert contained a sequence which began in the A portion and overlapped into the B region of each. The plasmids derived from the foregoing colonies are designated pRTB5, pRTB4, and pRTA115, respectively, herein.

4. The cDNA insert in pRTB5, which contained the coding sequence for the entire ricin B chain except for the 11 N-terminal amino acids, was excised and placed in the correct orientation with respect to the lac promoter by insertion into pUC8, to give pRTB151. pRTB151 was modified by the procedure described in paragraph C below to add the appropriate coding sequences, a start codon, and a conveniently placed upstream HindIII site to give pRTB601. The cloning vector used to obtain the cDNA library contains a HindIII site immediately downstream of the SalI site used for ligation into the vector, and thus the entire coding sequence including the start codon can be excised by treatment of the modified vector with HindIII.

Details of the strategy described above are set forth below.

## Gene Reconstruction by Tandem Bridging

In order to obtain the complete coding sequence for ricin B, including an additional start codon, a novel approach of general utility was employed. While devised to recreate the proper sequence for this particular protein, the techniques are applicable to any situation where cDNA library construction results in an incomplete copy of the coding sequence. They are also applicable even more generally to construction of a desired product double-stranded DNA sequence in a vector by modification of an available plasmid sequence.

The procedure is exemplified for specificity in terms of completing a missing sequence from the cDNA 5′ terminus. it could, of course, be used to complete either or both termini, to insert a desired sequence into the middle of a gene or to perform a variety of other modification reactions.

Briefly, for example, the cloning vector containing the desired cDNA insert is digested at a restriction site immediately preceding the coding sequences obtained. There will, of course, be such a restriction site if the appropriate linkers have been used to insert the cDNA fragment in the first place. Extra bases intervening between this restriction site and the beginning of the coding sequence are removed, if present, by treating with S1 nuclease. The resulting digested vector, then, has upstream and downstream cleavage termini, the downstream terminus being the 5′ end of the coding sequence and the upstream cleavage terminus being the sequences from the vector which were, before cleavage, immediately upstream of the cleavage site.

The two ends of the cleaved vector are then digested with exonuclease III. Exonuclease III hydrolizes nucleotide sequences which are in double-stranded status by digesting back beginning at the 3′ terminus of each of the strands. Thus, treatment of the digested cloning vector with exonuclease III will result in an exposed 5′ sticky end of the coding sequence at one end of the fragment and an exposed 5′ sticky end of the vector ligated upstream of the cDNA sequence at the other. It is not critical how far back into the cDNA or vector fragment the sticky end must extend, but 100 bases or more of single-stranded fragment portion is workable. At least two single-stranded oligonucleotides, but in any event an even number, are then supplied. The first of these nucleotides is, for the most part, complementary to the codons which would immediately precede the 5′ terminus of the cDNA codons, but also contains additional bases complementary to those in the exposed 5′ terminus of the insert. The oligonucleotide supplied preferably should contain sufficient extension past the 5′ terminus of the cDNA to pair with at least about 8 nucleotides.

The second oligonucleotide supplied should contain additional codons in the sense strand preceding those to which the first oligonucleotide is complementary. However, again, some area of overlap must be supplied to insure hybridization of the 3′ terminus of the second oligonucleotide to the 3′ terminus of the first. Additional single-stranded oligonucleotides may be supplied as necessary providing alternating additional complementary and coding sequences with overlapping regions.

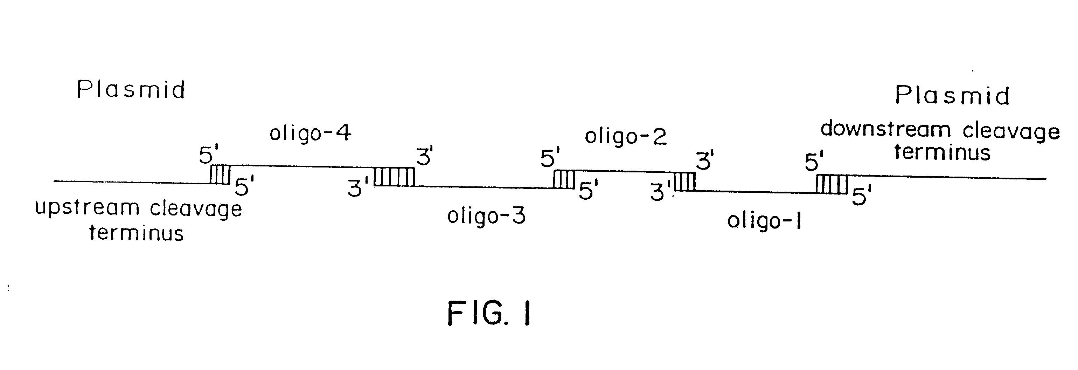

The strategy is best understood diagrammatically as outlined in Figure 1. As Figure 1 shows, the cDNA is extended by a series of bridging oligonucleotides in tandem, with complementarity ensuring hybridization between corresponding termini, ie, 5′ with 5′ and 3′ with 3′, a final oligonucleotide linking the bridge with the cloning vector 5′ sticky end.

The number of oligonucleotides required depends on the length of the sequence to be reconstructed. Each single-stranded oligonucleotide is more than about 18 bp in length and the regions of overlap at least about 8 bp at each terminus. The oligonucleotides are at least long enough so that when the overlap regions of complementarity are paired, at least 2 bases remain unpaired in the strand. If the reconstructed portion is, as in the illustration above, an N-terminus encoding sequence, an ATG start codon immediately preceding the codons of the desired sequence should be included and, optionally, for convenience, a restriction site preceding the ATG to permit easy manipulation of the resulting gene. If the codons to be replaced are at C-terminus, a stop codon should be included in appropriate reading frame, and can

conveniently be followed by a convenient restriction site sequence.

The exonuclease III treated vector is then mixed with the appropriate oligonucleotides and the mixture treated with both DNA polymerase I (Klenow) to fill in the complementary strands and with E. coli or other ligase to complete the construction. The vector now contains the extended cDNA insert to include the entire sequence, preceded, if desired, by a restriction site.

It will be apparent from the foregoing description that this tandem bridging technique is useful for the insertion of any desired sequence into a circular vector. As nucleotides can also be removed adjacent the original cleavage site, the end result may be modification as well as insertion.

## Improvements in mRNA Purification

In preparing the mRNA encoding ricin B, and its associated proteins, the method of Belamy, A. R, et al, Methods in Enzymology (1966) 104:156, was used with some modifications which effect improvements. These improvements and modifications overcome certain disadvantages of the standard methods and form the subject matter of copending European Patent Application No.  , which Application is also divided from European Patent Application No. 86301227.4. One such improvement succeeds in removing oxidized phenolic compounds from the preparations which constitute a problem in messenger RNA prepared from plant tissue sources. The other improvement has more general applicability in that it resolves a problem inherent in all mRNA preparation-ie, the tendency of mRNA to associate with ribosomal RNA. If this association can be destroyed before application of the preparation to the conventional dT affinity column, the requirements for elution are much less.

In order to eliminate oxidized phenolics, the preparation is treated with Sephadex G-100. The gel retains the oxidized phenolics and passes the mRNA in the void volume. (Both polyphenolic compounds and transfer RNA are retarded.) This offers a simpler solution than that conventionally used, ie, prior isolation of the ribosomes or other subcellular component before carrying out RNA extraction.

To eliminate the ribosomal RNA complexing, the procedure is modified so as to react the suspension of RNA emerging from the foregoing G-100 column, if such column is used, with a denaturant, or in any event so reacting it prior to applying the preparation to a dT affinity column. The preparation can be denatured with a minimal amount of a conventional denaturing agent such as, for example, heat, formamide, or methyl mercuric hydroxide, preferably formamide. The denaturing agent breaks down the association between the ribosomal RNA and polyA portions of the messenger RNA and thus permits the messenger to adhere to the column more effectively.

## Methods Employed

Isolation of the mRNA fragments comprising the desired coding sequences is described in detail herein below. The polyA RNA is used as a template to construct a cDNA library by means now well understood in the art. Details of such methods can be obtained by reference to Maniatis, E. F. et al, Molecular Cloning, Cold Spring Harbor Laboratory (1982). The cDNA library is probed for the desired sequences using the procedure of Grunstein and Hogness, Proc Natl Acad Sci (1975) 72:3961.

Vector construction employs ligation and restriction techniques known in the art. The quantity of DNA available can be increased by cloning the desired fragments, ie, inserting into a suitable cloning vehicle, such as pBR322, pUC13 or pUC8, transforming and replicating in E. coli, and, optionally further enhancing through chloramphenicol amplification or by phage replication. The desired fragments can then be removed from the cloning vectors or phage and ligated to suitable promoters compatible with the host intended to be employed in the expression of the gene. Such hosts are then transformed with these expression vectors and cultured under conditions which favor stabilization of the plasmid and the safe production of the desired toxin fragments. Such conditions might include repression of the controlling promoter until most of log phase growth has been completed, and then altering conditions so as to favor the synthesis of the peptide. When the peptide has been synthesized, the cells are lysed, and the desired fragment recovered from the lysate.

Construction of suitable vectors containing the desired coding and control sequences employs standard ligation and restriction techniques which are well understood in the art. Isolated plasmids, DNA sequences, or synthesized oligonucleotides are cleaved, tailored, and religated in the form desired.

Site specific DNA cleavage is performed by treating with the suitable restriction enzyme (or enzymes) user conditions which are generally understood in the art, and the particulars of which are specified by the

manufacturer of these commercially available restriction enzymes. See, eg, New England Biolabs, Product Catalog. In general, about 1 µg of plasmid or DNA sequence is cleaved by one unit of enzyme in about 20 µl of buffer solution; in the examples herein, typically, an excess of restriction enzyme is used to insure complete digestion of the DNA substrate. Incubation times of about one hour to two hours at about 37° C are workable, although variations can be tolerated. After each incubation, protein is removed by extraction with phenol/chloroform followed by ether extraction and the nucleic acid is recovered from aqueous fractions by precipitation with ethanol followed by running over a Sephadex G-50 spin column. If desired, size separation of the cleaved fragments may be performed by polyacrylamide gel electrophoresis using standard techniques. A general description of size separations is found in Methods in Enzymology (1980) 65:499-560.

Restriction cleaved fragments may be blunt ended by treating with the large fragment of E. coli DNA polymerase I (Klenow) in the presence of the four nucleotide triphosphates (dNTPs) using incubation times of about 15 to 25 min at 20 to 25° C in 50 mM Tris pH 7.6, 50 mM NaCl, 6 mM $MgCl_2$, 6 mM DTT and 0.1 mM dNTPS. The Klenow fragment fills in at 5′ sticky ends but chews back single strands, even though the four dNTPs are present, at 3′ sticky ends. If desired, selective repair can be performed by supplying only one of the, or selected, dNTPs within the limitations dictated by the nature of the sticky ends. After treatment with Klenow, the mixture is extracted with phenol/chloroform and ethanol precipitated followed by running over a Sephadex G-50 spin column.

Two other nuclease enzymes are used in the procedures set forth below: S1 nuclease and exonuclease III.

Treatment with S1 nuclease under appropriate conditions results in rapid hydrolysis of any single-stranded portion of DNA and slow hydrolysis of double-stranded portions commencing at the ends. S1 nuclease hydrolyses are conducted in a buffer which is 15 mM sodium acetate, pH 4.5, 200 mM NaCl, and 1 mM $ZnSO_4$, using approximately 200 units per µl of S1 nuclease. Ordinarily, 5000 units of S1 nuclease is used to hydrolyze approximately 10 µg of DNA.

Exonuclease III attacks double-stranded DNA, but hydrolyzes beginning at the 3′ end of the nucleotide sequence. Thus, digestion of a double-stranded DNA results in two 5′ protruding sticky ends. Hydrolysis is carried out in a buffer containing 15 mM Tris, pH 8, 10 mM NaCl, 1 mM $MgCl_2$, and 0.1 mM DTT, using approximately 2000 units per µl exonuclease III. Ordinarily, 150 units of exonuclease III were used to react with 10 µg DNA.

Synthetic oligonucleotides ace prepared by the triester method of Matteucci, et al (J Am Chem Soc (1981) 103:3185-3191). Kinasing of single strands prior to annealing or for labeling is achieved using an excess, e.g., approximately 10 units of polynucleotide kinase to 1 nmole substrate in the presence of 50 mM Tris, pH 7.6, 10 mM $MgCl_2$, 5 mM dithiothreitol, 1-2 mM ATP, 1.7 pmoles $\gamma^{32}$ P ATP (2.9 mCi/mmole), 0.1 mM spermidine, 0.1 mM EDTA.

Ligations are formed using approximately equimolar amounts of the desired DNA fragments (2-10 x excess of linkers or small oligomers) suitably end tailored to provide correct matching, by treatment with an excess, i.e., in a typical 15-30 µl reaction 0.4-4 Weiss units T4 DNA ligase and, when blunt-ended ligation is involved, 0.4-1 units of RNA ligase. Ligation mixtures are buffered at approximately pH 7.6 using 66 mM Tris along with 5 mM magnesium ion, 5 mM dithiothreitol, 1 mM ATP, and 0.1 mg/ml BSA for either blunt-end or sticky end ligations. Incubations are carried out at approximately 14 to 25° C overnight.

In vector construction employing "vector fragments," the vector fragment is sometimes treated with bacterial alkaline phosphatase (BAP) in order to remove the 5′ phosphate and prevent religation of the vector. BAP digestions are conducted at pH 8.3 in approximately 50 mM Tris, in the presence of $Na^+$ and $Mg^{+2}$ using about 1 unit of BAP per µg of vector at 60° for about one hour in order to recover the nucleic acid fragments, the preparation is extracted with phenol chloroform and ethanol precipitated and desalted by application to a Sephadex G-50 spin column. Alternatively, religation can be prevented in vectors which have been double digested by additional restriction enzyme cleavage of the unwanted fragments.

In the constructions set forth below, correct ligations for plasmid construction are confirmed by transforming E. coli strain MM294 obtained from E. coli Genetic Stock Center CGSC #6135, or other suitable host with the ligation mixture. Successful transformants are selected by ampicillin, tetracycline or other antibiotic resistance or using other markers depending on the mode of plasmid construction, as is understood in the art. Plasmids from the transformants are then prepared according to the method of Clewell, D. B., et al, Proc Natl Acad Sci (1969) 62:1159, following chloramphenicol amplification (Clewell, D. B., J Bacteriol (1972) 110:667) and analyzed by restriction and/or sequenced by the method of Messing, et al, Nucleic Acids Res (1981) 9:309, or by the method of Maxam, et al, Methods in Enzymology (1980) 65:499.

Transformations in the examples below were performed using the calcium chloride method described

by Cohen, S. N., et al, Proc Natl Acad Sci (USA) (1972) 69:2110.

For cloning and sequencing, E. coli strain MM294 (supra), Talmadge, K., et al, Gene (1980) (12:235; Meselson, M., et al, Nature (1968) 217:1110, was used as the host.

The following Example illustrates the invention by describing the production of a ricin B encoding cassette.

## EXAMPLE

### Isolation of Messenger RNA and Preparation of a cDNA Library

#### 1 Isolation of mRNA from Castor Beans

50 g castor beans (Ricinus communis) were placed in 100 ml homogenzizing buffer (150 mM NaCl, 50 mM Tris, pH 8.3, 5 mM EDTA AND 50 mM freshly added β-mercaptoethanol) to which was added 12 ml 0.2 M vanadium-ribonucleoside complex,* 30 mg proteinase K, and 15 ml 20% SDS. The mixture was homogenized by blending at high speed for 3-4 min in a Waring blender and then incubating 2-3 hr at room temperature with occasional blending.

(*The vanadium ribonucleoside solution is prepared as follows: 893 mg $VOSO_4 . 3H_2O$ was added to 2 ml water and the mixture boiled to dissolve the vanadium salt. A solution containing the 4 ribonucleosides was prepared by dissolving 1 mmole each of adenosine, cytidine, guanosine, and uridine in 17 ml water. Heat is required. 1 ml of the foregoing $VOSO_4$ solution was then added to the ribonucleoside solution and the resultant titrated to pH 6.5 with 10 N NaOH, and finally to pH 7 with 1 N NaOH while stirring in a boiling water bath. Formation of the complex is indicated by a change in color from bright blue to green-black. The solution was finally diluted to 20 ml with water.)

The suspension was centrifuged for 15 min at 8000 x g at 5° C and the pellet discarded. The supernatant was strained through cheesecloth to remove lipids and the filtrate extracted sufficiently with phenol:$CHCl_3$:isoamyl alcohol, 24:24:1 containing 1% hydroxy-quinoline, to remove vanadium salts and protein, and the aqueous layer brought to 0.4 M with NaCl and 10 mM with EDTA. 2.5 x volume absolute ethanol was added to precipitate nucleic acids, and the mixture stored at -20° C overnight.

The precipitate was centrifuged for 15 min at 7000 x g at 2° C, and the pellet resuspended in 9.5 ml aqueous solution 0.025 M NaCl, 0.025 M Tris, pH 8, plus 9.5 ml phosphate buffer (2.5 M total phosphate, $K_2HPO_4$:33% $H_3PO_4$, 20:1), plus 9.5 ml 2-methoxyethanol. The mixture was shaken and chilled on ice for 3-5 min with occasional mixing, and then centrifuged at 2000 x g for 5 min at 2° C. The upper layer was removed and to this was added 10 ml 0.2 M sodium acetate, and 5 ml 1% cetyl trimethylammonium bromide (CTAB) and the mixture chilled on ice for 10 min. The resulting white precipitate was harvested by centrifugation at 2000 x g for 10 min at 2° C. The precipitate was washed by addition of 70% ethanol in 0.1 M sodium acetate and by re-centrifuging at 2500 x g for 10 min at 4° C.

After removal of the supernatant, the pellet was resuspended in 2 ml G-100 column starting buffer (20 mM Tris, pH 8, 1 mM EDTA, 0.5% SDS), and then adjusted to contain 0.5 M NaCl. Solids were removed by centrifugation at 2000 xg for 5 min at room temperature and the supernatant applied to a Sephadex G-100 column (1.5 cm x 40 cm) and the column eluted using buffer similar to that applied to the column but lacking SDS. The eluate was assayed by monitoring $OD_{260}$. Desired messenger RNA was obtained in the flow through volume, leaving behind oxidized phenolic compounds present in the plant extract. (These compounds are known to behave similarly to polyA RNA on dT columns, inhibit protein synthesis, and thus interfere with the assay for mRNA.)

The initial peak containing mRNA was treated with formamide, a denaturant, to destroy ribosomal RNA complexing. To do this, the mRNA containing fractions were pooled, precipitated in ethanol, and the precipitates redissolved in a minimum volume of water. To this solution was added 9 volumes of deionized formamide containing 20 mM PIPES (piperazine-N,N-bis(2-ethanesulfonic acid), pH 6.5-7.0.

The mixture was then warmed to 37° C for 5 min, and 10 volumes of dT column buffer (0.5 M NaCl, I0 mM Tris, pH 7.5, 1 mM EDTA) added. The presence of formamide dissociates the polyA RNA from any ribosomal RNA present.

The denatured mixture was then run over an oligo dT column according to procedures well established

in the art, and approximately 100 µg polyA RNA recovered upon elution.

## 2 Formation of a cDNA Library

The polyA mRNA prepared as in the preceding paragraph was used to obtain a cDNA library according to the method of Maniatis, et al (supra). Briefly, a portion of the polyA RNA is treated under appropriate buffer conditions with reverse transcriptase and then treated with base to destroy the remaining mRNA. The resulting single-stranded cDNA is repaired using E. coli Polymerase I (Klenow fragment) in the presence of the 4 dNTPs and the resulting "hairpin" then ligated using T4 ligase to a SalI linker (obtained from New England BioLabs). After treating with S1 nuclease and repairing with Klenow, the blunt end was ligated to an EcoRI linker using $T_4$ ligase. After then digesting with EcoRI and SalI, the resulting double-stranded cDNA fragments, which are bounded by EcoRI and SalI restriction sites, were ligated into a EcoRI/SalI digested, BAP treated preparation of pUC13 obtained and freely available from J. Messing, the University of Minnesota. pUC13 is a modification of pBR322 capable of confering Amp resistance ($Amp^R$), which contains linkers bearing convenient restriction sites, including a PstI site downstream from the SalI site used in the insertion. The resulting ligation mixture was used to transform E. coli MM294, and $Amp^R$ strains selected.

Successful colonies were transferred onto nitrocellulose plates, and probed using the procedure of Grunstein & Hogness (supra), with the mixture of 16 synthetic oligonucleotides

5′ CC($^{A}_{G}$)TC($^{A}_{G}$)TT($^{C}_{T}$)TT($^{A}_{G}$)AACATCC 3′

which is kinased with $^{32}P$. This mixture represents the anti-sense strand complementary to the codons for the amino acid sequence Trp-Met-Phe-Lys-Asn-Asp-Gly. Of about 5000 colonies probed about 1% were found which hybridized to the probe. Plasmids were isolated from several representations of these colonies, and analyzed by restriction analysis and Maxam-Gilbert sequencing. Three plasmids, pRTB4, pRTB5, and pRTA115 were sequenced in the insert region.

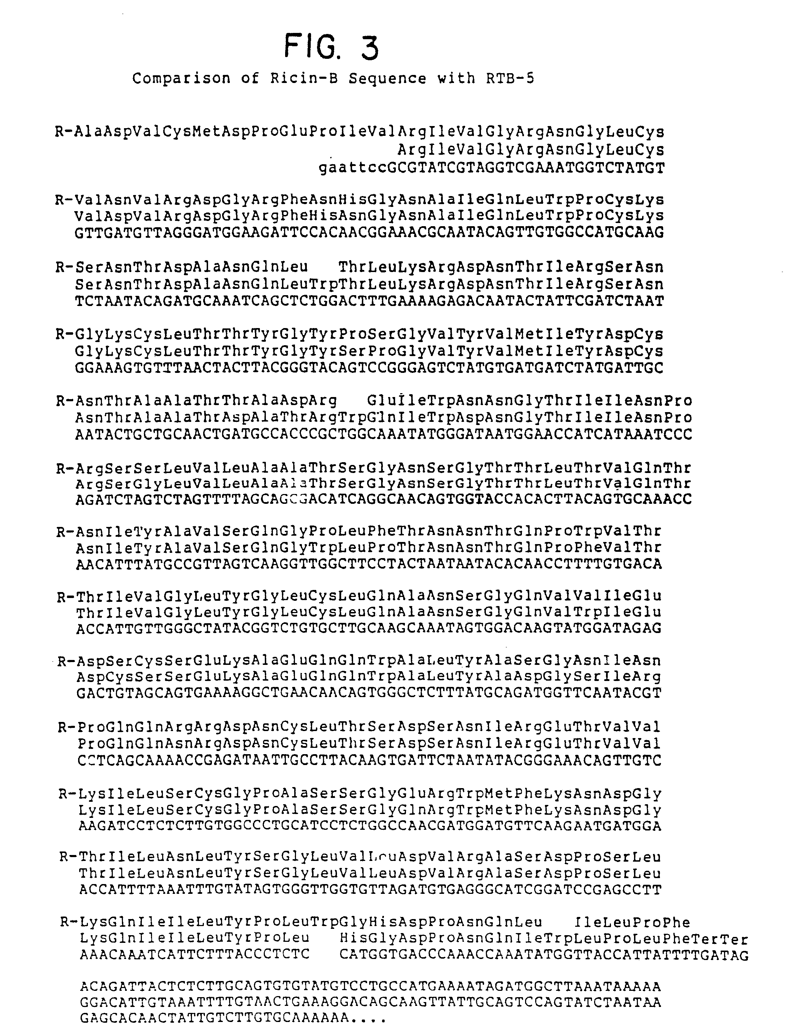

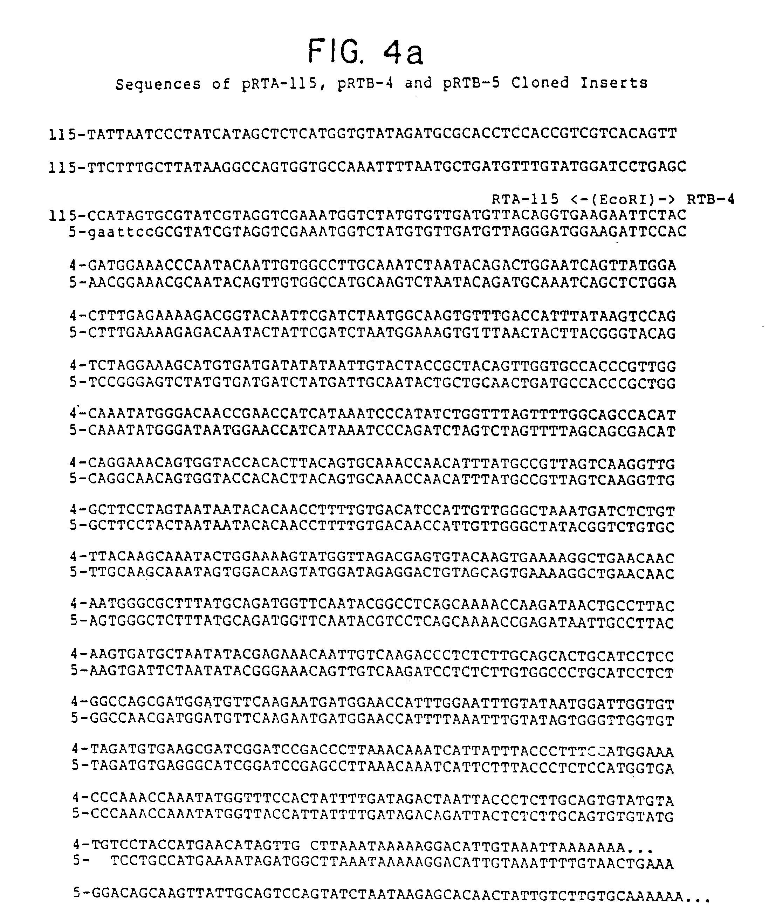

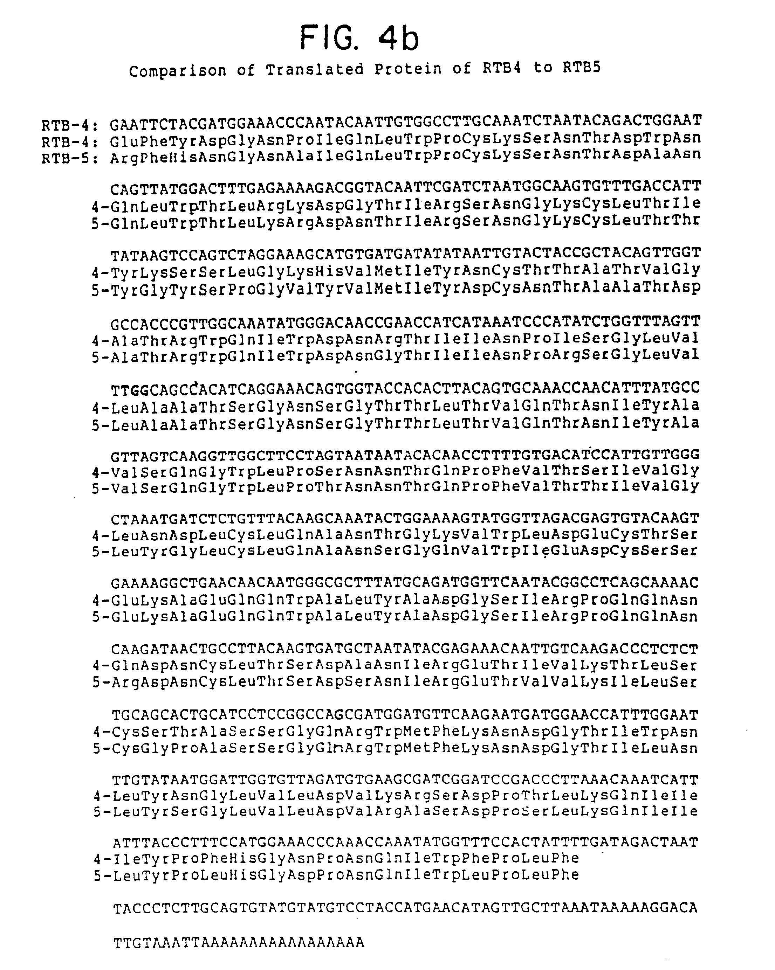

Figures 3 and 4 show the results of this sequencing. Figure 3 shows the sequence of the insert in pRTB5. Line 1 in Figure 3 represents the amino acid sequence of ricin B as determined by Funatsu (supra). The second line represents the amino acid sequence deduced from the pRTB5 base sequence. An examination of the deduced sequence shows a high level of correspondence, although some discrepancies exist. These are due to errors in the published sequence and to varietal differences in the ricin B proteins represented. Line 3 is the base sequence of the isolated cDNA. The entire coding sequence for ricin B is present except for codons for the first 11 amino acids. (The lower case codons at the 5′ end represent the EcoRI linker used to ligate the cDNA insert into the plasmid.) pRTB5 was used as the source of the bulk of the coding sequence.

Figure 4 shows a comparison of the sequences in pRTB4 and pRTB5. It is believed that the pRTB4 sequence represents the coding for RCA B chain. Figure 4 also shows the overlap between pRTA115 and pRTB5 which indicates that the RTA115 insert contains the upstream coding regions of the ricin B gene. Although pRTA115 is believed associated with the RCA precursor protein, the amino acid sequence deduced from pRTA115 for RCA matches that of ricin B for the 11 amino acids needed to complete the N-terminus. These sequences were therefore used as models for the construction of oligonucleotides encoding the missing 11 N-terminus codons and also permit the deduction of the amino acid sequence of the 12 amino acid peptide in the single peptide precursor of RCA and, presumably, of ricin A and B.

The coding sequences of pRTB5 were disposed so as not to be expressible under the control of the lac promoter as inserted into pUC13. Therefore pRTB5 was cut with EcoRI and PstI and the vector cleaved into several fragments with BstNI. The insert fragment was ligated under standard conditions using T4 ligase with an EcoRI/PstI digest of pUC8, another modified pBR322 vector obtained from and freely available from Messing, J., at the University of Minnesota. pUC8 has EcoRI and PstI sites which place an EcoRI/PstI insert under lac promoter control as a fusion protein with the initial 5-8 amino acids of β-galactosidase. It also contains a HindIII site immediately downstream from the PstI site. The ligation mixture was transformed into E. coli MM294, and transformants selected for ampicillin resistance. Plasmid DNA was isolated from successful transformants in several colonies, and analyzed by restriction site mapping. Colonies showing the appropriate restriction patterns were selected. One colony, designated pRTB151, was tested for expression of the gene for the fusion protein. On Western Blot no protein band corresponding to the desired molecular weight was found, although cross-reacting proteins were produced. It was assumed that the reading frame might be improper, since this plasmid was designed to have the β-galactosidase and ricin B sequences in different phases.

Construction of the Ricin B Coding Sequence as a HindIII-Cassette - pRTB601

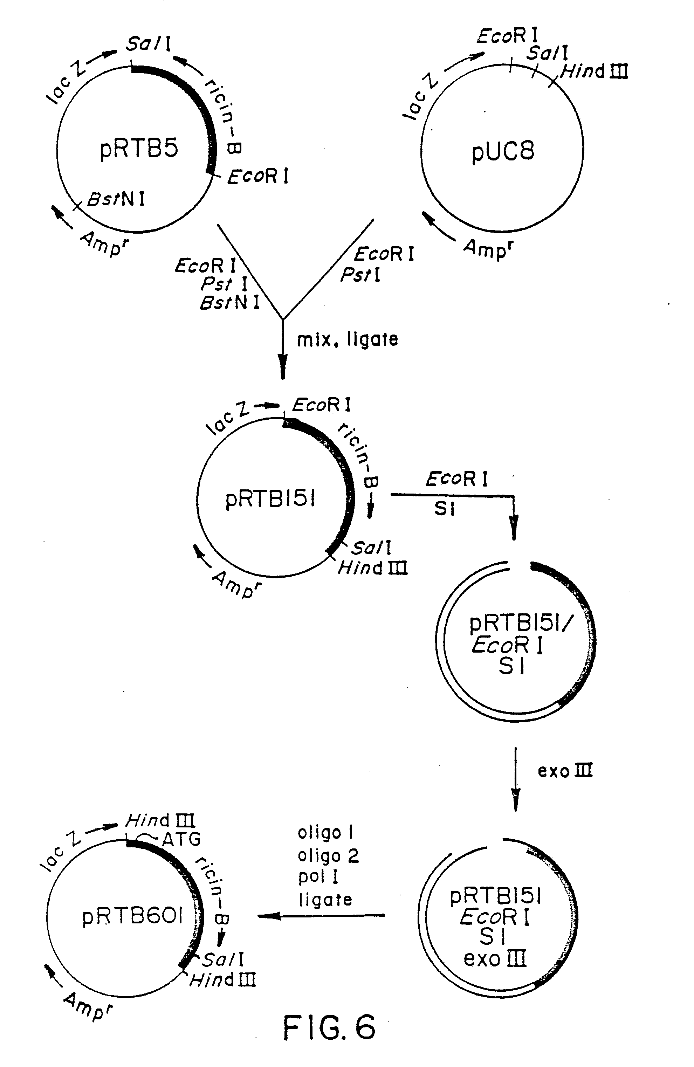

The construction is outlined in Figure 6.

Ten µg of pRTB151 DNA was digested to completion with EcoRI, dissolved in 60 µl S1 buffer and digested for 4 min at room temperature under conditions which remove about 1 base pair of duplex DNA per min. DNA recovered from the foregoing buffer was dissolved in 60 µl exonuclease III buffer and digested for 4 min at room temperature. Subsequent analysis showed that the plasmid DNA had lost approximately 120 bp from each 3′ end, leaving 5′ ends available for hybridization. DNA recovered from the Exonuclease III buffer was dissolved in 50 µl water and 20 µl used in the ligation/repair reaction below.

Thus, 20 µl sample (2 pmoles) was mixed with 20 pmoles each of the synthetic oligonucleotides:

```
                        Oligo 2
5'- GACCATGATAAGCTTATGGCTGATGTTTGTATGGATCC and
               HindIII     3'TACCTAGGACTCGGGTATCACGCATAGCATCC-5'
                                                Oligo 1
```

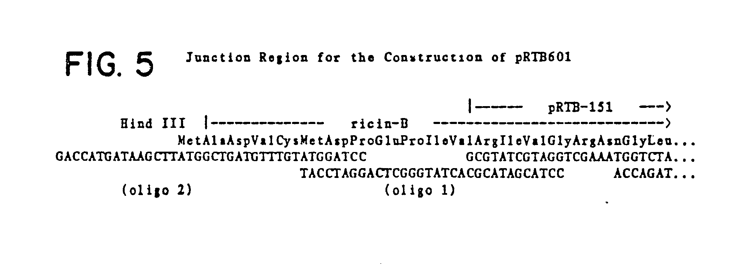

which have complementary sequences as shown, and wherein Oligo-2 encodes a HindIII site upstream of an ATG start codon as shown in Figure 5. The 5′ end of Oligo-1 is complementary to 15 bases at the 5′ end of the pRTB151 cDNA sequence as there shown and is complementary to the contiguous missing codons of the ricin B sequence. The 5′ end of Oligo-2 is complementary to the 5′ sticky end of the vector residue of the exonuclease III treated pRTB151.

The mixture was heated to 60° C for 5 min in order to denature completely complementation of single-stranded DNA, cooled to 37° C for 5 min to hybridized complementary strands, and then chilled on ice. The soluton was brought to polymerase I (Klenow) buffer conditions and reacted for 2 hr at l2° C in the presence of the 50 µM each of the 4 dNTPs, 0.1 mM NAD, 0.3 units/µl Klenow, and 0.08 units/µl E. coli DNA ligase. The ligation mixture was used directly to transform competent E. coli MM294 and several thousand $Amp^R$ colonies found. Several hundred of these were replicated and grown on nitrocellulose filters and subjected to standard colony hybridization using $P^{32}$ kinased Oligo-2 as probe. Two clones which hybridized with the probe were analyzed by restriction analysis and sequenced, and a correct construction designated pRTB601. pRTB601 thus contains the ricin B coding sequence as a HindIII cassette. The upstream HindIII site is introduced immediately upstream of the ATG codon in Oligo-2; the downstream HindIII site arises from the pUC8 vector plasmid.

## Claims

1. A method of modifying the DNA sequence in a circular vector to give a desired circular DNA sequence which method comprises:

(a) treating the circular vector with a restriction enzyme to obtain a linear vector having an upstream cleavage terminus and a downstream cleavage terminus;

(b) optionally treating the linear vector of (a) to remove undesired bases from the cleavage termini;

(c) treating the linear vector of (a) or (b) with exonuclease III to generate 5′ protruding sticky ends extending at least about 10 bases at each of the upstream and downstream cleavage termini;

(d) mixing the resultant of (c) with an even number of single-stranded oligonucleotides, each oligonucleotide containing at least about 18 bases, wherein said oligonucleotides are comprised alternately of anti-sense and sense sequences of the desired circular DNA sequence which are missing from, and contiguous, in the desired DNA circular sequence, to the DNA sequence of the linear vector of (c). and wherein

(i) the first oligonucleotide is an anti-sense strand and has a 5′ terminus which overlaps so as to be complementary to at least about 8 bases of the protruding 5′ sticky end of the downstream cleavage terminus;

(ii) the last numbered oligonucleotide is a sense strand and has a 5′ terminus which overlaps so as to be complementary to at least about 8 bases of the 5′ protruding sticky end of the upstream cleavage terminus; and

(iii) each of the remaining (second through second to last) oligonucleotides are alternately sense and anti-sense strands each having a 5′ terminus which overlaps with so as to be complementary to the 5′ terminus of the adjacent oligonucleotide, and a 3′ terminus which overlaps with so as to be complementary to the 3′ terminus of the adjacent oligonucleotide;

(iv) wherein the oligonucleotides are sufficiently long so that the regions of overlap leave single stranded portions of at least about 2 bases; and

(e) treating the mixture of (c) with a DNA polymerase and a ligase.

2. The method of claim 1 wherein the desired circular DNA sequence is the DNA sequence of the starting circular vector containing the additional sequences of the oligonucleotides.

3. The method of claim 2 wherein the desired circular DNA sequence includes the complete DNA sequence encoding a desired protein and the starting circular DNA sequence includes a cDNA insert encoding said desired protein less a sequence encoding the N-terminus of the desired protein.

4. The method of claim 3 wherein the step (a) utilizes a restriction enzyme which cleaves proximal to the 5′ terminus of the cDNA insert.

5. The method of claim 3 wherein the downstream cleavage terminus is proximal to the 5′ terminus of the cDNA insert.

6. The method of claim 5 wherein the last numbered oligonucleotide encodes, in proper reading frame with the codons for the desired protein, an ATG start codon.

7. The method of claim 6 wherein the last numbered oligonucleotide further includes a restriction site upstream from the ATG.

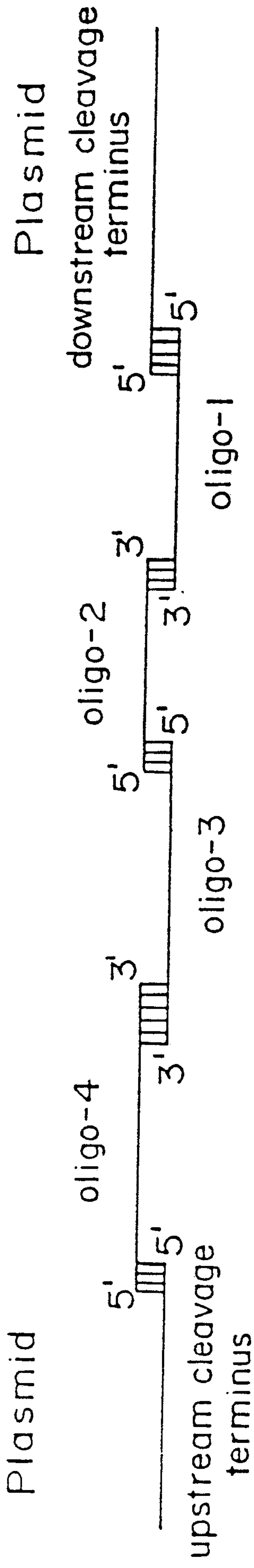

Plasmid
upstream cleavage terminus
5'
5'
oligo-4
3'
3'
oligo-3
5'
5'
oligo-2
3'
3'
oligo-1
5'
5'
Plasmid
downstream cleavage terminus


FIG. I

# FIG. 2

Ricin-B Protein Sequence

AlaAspValCysMetAspProGluProIleValArgIleValGlyArgAsnGlyLeuCys
ValAsnValArgAspGlyArgPheAsnHisGlyAsnAlaIleGlnLeuTrpProCysLys
SerAsnThrAspAlaAsnGlnLeuThrLeuLysArgAspAsnThrIleArgSerAsnGly
LysCysLeuThrThrTyrGlyTyrProSerGlyValTyrValMetIleTyrAspCysAsn
ThrAlaAlaThrThrAlaAspArgGluIleTrpAsnAsnGlyThrIleIleAsnProArg
SerSerLeuValLeuAlaAlaThrSerGlyAsnSerGlyThrThrLeuThrValGlnThr
AsnIleTyrAlaValSerGlnGlyProLeuPheThrAsnAsnThrGlnProTrpValThr
ThrIleValGlyLeuTyrGlyLeuCysLeuGlnAlaAsnSerGlyGlnValValIleGlu
AspSerCysSerGluLysAlaGluGlnGlnTrpAlaLeuTyrAlaSerGlyAsnIleAsn
ProGlnGlnArgArgAspAsnCysLeuThrSerAspSerAsnIleArgGluThrValVal
LysIleLeuSerCysGlyProAlaSerSerGlyGluArgTrpMetPheLysAsnAspGly
ThrIleLeuAsnLeuTyrSerGlyLeuValLeuAspValArgAlaSerAspProSerLeu
LysGlnIleIleLeuTyrProLeuTrpGlyHisAspProAsnGlnLeuIleLeuProPhe

(260 a.a -- from Funatsu)

# FIG. 3

Comparison of Ricin-B Sequence with RTB-5

```
R-AlaAspValCysMetAspProGluProIleValArgIleValGlyArgAsnGlyLeuCys
                                      ArgIleValGlyArgAsnGlyLeuCys
                               gaattccGCGTATCGTAGGTCGAAATGGTCTATGT

R-ValAsnValArgAspGlyArgPheAsnHisGlyAsnAlaIleGlnLeuTrpProCysLys
  ValAspValArgAspGlyArgPheHisAsnGlyAsnAlaIleGlnLeuTrpProCysLys
  GTTGATGTTAGGGATGGAAGATTCCACAACGGAAACGCAATACAGTTGTGGCCATGCAAG

R-SerAsnThrAspAlaAsnGlnLeu   ThrLeuLysArgAspAsnThrIleArgSerAsn
  SerAsnThrAspAlaAsnGlnLeuTrpThrLeuLysArgAspAsnThrIleArgSerAsn
  TCTAATACAGATGCAAATCAGCTCTGGACTTTGAAAAGAGACAATACTATTCGATCTAAT

R-GlyLysCysLeuThrThrTyrGlyTyrProSerGlyValTyrValMetIleTyrAspCys
  GlyLysCysLeuThrThrTyrGlyTyrSerProGlyValTyrValMetIleTyrAspCys
  GGAAAGTGTTTAACTACTTACGGGTACAGTCCGGGAGTCTATGTGATGATCTATGATTGC

R-AsnThrAlaAlaThrThrAlaAspArg   GluIleTrpAsnAsnGlyThrIleIleAsnPro
  AsnThrAlaAlaThrAspAlaThrArgTrpGlnIleTrpAspAsnGlyThrIleIleAsnPro
  AATACTGCTGCAACTGATGCCACCCGCTGGCAAATATGGGATAATGGAACCATCATAAATCCC

R-ArgSerSerLeuValLeuAlaAlaThrSerGlyAsnSerGlyThrThrLeuThrValGlnThr
  ArgSerGlyLeuValLeuAlaAlaThrSerGlyAsnSerGlyThrThrLeuThrValGlnThr
  AGATCTAGTCTAGTTTTAGCAGCGACATCAGGCAACAGTGGTACCACACTTACAGTGCAAACC

R-AsnIleTyrAlaValSerGlnGlyProLeuPheThrAsnAsnThrGlnProTrpValThr
  AsnIleTyrAlaValSerGlnGlyTrpLeuProThrAsnAsnThrGlnProPheValThr
  AACATTTATGCCGTTAGTCAAGGTTGGCTTCCTACTAATAATACACAACCTTTTGTGACA

R-ThrIleValGlyLeuTyrGlyLeuCysLeuGlnAlaAsnSerGlyGlnValValIleGlu
  ThrIleValGlyLeuTyrGlyLeuCysLeuGlnAlaAsnSerGlyGlnValTrpIleGlu
  ACCATTGTTGGGCTATACGGTCTGTGCTTGCAAGCAAATAGTGGACAAGTATGGATAGAG

R-AspSerCysSerGluLysAlaGluGlnGlnTrpAlaLeuTyrAlaSerGlyAsnIleAsn
  AspCysSerSerGluLysAlaGluGlnGlnTrpAlaLeuTyrAlaAspGlySerIleArg
  GACTGTAGCAGTGAAAAGGCTGAACAACAGTGGGCTCTTTATGCAGATGGTTCAATACGT

R-ProGlnGlnArgArgAspAsnCysLeuThrSerAspSerAsnIleArgGluThrValVal
  ProGlnGlnAsnArgAspAsnCysLeuThrSerAspSerAsnIleArgGluThrValVal
  CCTCAGCAAAACCGAGATAATTGCCTTACAAGTGATTCTAATATACGGGAAACAGTTGTC

R-LysIleLeuSerCysGlyProAlaSerSerGlyGluArgTrpMetPheLysAsnAspGly
  LysIleLeuSerCysGlyProAlaSerSerGlyGlnArgTrpMetPheLysAsnAspGly
  AAGATCCTCTCTTGTGGCCCTGCATCCTCTGGCCAACGATGGATGTTCAAGAATGATGGA

R-ThrIleLeuAsnLeuTyrSerGlyLeuValLeuAspValArgAlaSerAspProSerLeu
  ThrIleLeuAsnLeuTyrSerGlyLeuValLeuAspValArgAlaSerAspProSerLeu
  ACCATTTTAAATTTGTATAGTGGGTTGGTGTTAGATGTGAGGGCATCGGATCCGAGCCTT

R-LysGlnIleIleLeuTyrProLeuTrpGlyHisAspProAsnGlnLeu   IleLeuProPhe
  LysGlnIleIleLeuTyrProLeu   HisGlyAspProAsnGlnIleTrpLeuProLeuPheTerTer
  AAACAAATCATTCTTTACCCTCTC   CATGGTGACCCAAACCAAATATGGTTACCATTATTTGATAG

  ACAGATTACTCTCTTGCAGTGTGTATGTCCTGCCATGAAAATAGATGGCTTAAATAAAAA
  GGACATTGTAAATTTTGTAACTGAAAGGACAGCAAGTTATTGCAGTCCAGTATCTAATAA
  GAGCACAACTATTGTCTTGTGCAAAAAA....
```

# FIG. 4a

Sequences of pRTA-115, pRTB-4 and pRTB-5 Cloned Inserts

115-TATTAATCCCTATCATAGCTCTCATGGTGTATAGATGCGCACCTCCACCGTCGTCACAGTT

115-TTCTTTGCTTATAAGGCCAGTGGTGCCAAATTTTAATGCTGATGTTTGTATGGATCCTGAGC

RTA-115 <-(EcoRI)-> RTB-4
115-CCATAGTGCGTATCGTAGGTCGAAATGGTCTATGTGTTGATGTTACAGGTGAAGAATTCTAC
5-gaattccGCGTATCGTAGGTCGAAATGGTCTATGTGTTGATGTTAGGGATGGAAGATTCCAC

4-GATGGAAACCCAATACAATTGTGGCCTTGCAAATCTAATACAGACTGGAATCAGTTATGGA
5-AACGGAAACGCAATACAGTTGTGGCCATGCAAGTCTAATACAGATGCAAATCAGCTCTGGA

4-CTTTGAGAAAAGACGGTACAATTCGATCTAATGGCAAGTGTTTGACCATTTATAAGTCCAG
5-CTTTGAAAAGAGACAATACTATTCGATCTAATGGAAAGTGTTTAACTACTTACGGGTACAG

4-TCTAGGAAAGCATGTGATGATATATAATTGTACTACCGCTACAGTTGGTGCCACCCGTTGG
5-TCCGGGAGTCTATGTGATGATCTATGATTGCAATACTGCTGCAACTGATGCCACCCGCTGG

4-CAAATATGGGACAACCGAACCATCATAAATCCCATATCTGGTTTAGTTTTGGCAGCCACAT
5-CAAATATGGGATAATGGAACCATCATAAATCCCAGATCTAGTCTAGTTTTAGCAGCGACAT

4-CAGGAAACAGTGGTACCACACTTACAGTGCAAACCAACATTTATGCCGTTAGTCAAGGTTG
5-CAGGCAACAGTGGTACCACACTTACAGTGCAAACCAACATTTATGCCGTTAGTCAAGGTTG

4-GCTTCCTAGTAATAATACACAACCTTTTGTGACATCCATTGTTGGGCTAAATGATCTCTGT
5-GCTTCCTACTAATAATACACAACCTTTTGTGACAACCATTGTTGGGCTATACGGTCTGTGC

4-TTACAAGCAAATACTGGAAAAGTATGGTTAGACGAGTGTACAAGTGAAAAGGCTGAACAAC
5-TTGCAAGCAAATAGTGGACAAGTATGGATAGAGGACTGTAGCAGTGAAAAGGCTGAACAAC

4-AATGGGCGCTTTATGCAGATGGTTCAATACGGCCTCAGCAAAACCAAGATAACTGCCTTAC
5-AGTGGGCTCTTTATGCAGATGGTTCAATACGTCCTCAGCAAAACCGAGATAATTGCCTTAC

4-AAGTGATGCTAATATACGAGAAACAATTGTCAAGACCCTCTCTTGCAGCACTGCATCCTCC
5-AAGTGATTCTAATATACGGGAAACAGTTGTCAAGATCCTCTCTTGTGGCCCTGCATCCTCT

4-GGCCAGCGATGGATGTTCAAGAATGATGGAACCATTTGGAATTTGTATAATGGATTGGTGT
5-GGCCAACGATGGATGTTCAAGAATGATGGAACCATTTTAAATTTGTATAGTGGGTTGGTGT

4-TAGATGTGAAGCGATCGGATCCGACCCTTAAACAAATCATTATTTACCCTTTCCATGGAAA
5-TAGATGTGAGGGCATCGGATCCGAGCCTTAAACAAATCATTCTTTACCCTCTCCATGGTGA

4-CCCAAACCAAATATGGTTTCCACTATTTTGATAGACTAATTACCCTCTTGCAGTGTATGTA
5-CCCAAACCAAATATGGTTACCATTATTTTGATAGACAGATTACTCTCTTGCAGTGTGTATG

4-TGTCCTACCATGAACATAGTTG CTTAAATAAAAAGGACATTGTAAATTAAAAAAA...
5- TCCTGCCATGAAAATAGATGGCTTAAATAAAAAGGACATTGTAAATTTGTAACTGAAA

5-GGACAGCAAGTTATTGCAGTCCAGTATCTAATAAGAGCACAACTATTGTCTTGTGCAAAAAA...

# FIG. 4b

Comparison of Translated Protein of RTB4 to RTB5

```
RTB-4: GAATTCTACGATGGAAACCCAATACAATTGTGGCCTTGCAAATCTAATACAGACTGGAAT
RTB-4: GluPheTyrAspGlyAsnProIleGlnLeuTrpProCysLysSerAsnThrAspTrpAsn
RTB-5: ArgPheHisAsnGlyAsnAlaIleGlnLeuTrpProCysLysSerAsnThrAspAlaAsn

       CAGTTATGGACTTTGAGAAAAGACGGTACAATTCGATCTAATGGCAAGTGTTTGACCATT
     4-GlnLeuTrpThrLeuArgLysAspGlyThrIleArgSerAsnGlyLysCysLeuThrIle
     5-GlnLeuTrpThrLeuLysArgAspAsnThrIleArgSerAsnGlyLysCysLeuThrThr

       TATAAGTCCAGTCTAGGAAAGCATGTGATGATATATAATTGTACTACCGCTACAGTTGGT
     4-TyrLysSerSerLeuGlyLysHisValMetIleTyrAsnCysThrThrAlaThrValGly
     5-TyrGlyTyrSerProGlyValTyrValMetIleTyrAspCysAsnThrAlaAlaThrAsp

       GCCACCCGTTGGCAAATATGGGACAACCGAACCATCATAAATCCCATATCTGGTTTAGTT
     4-AlaThrArgTrpGlnIleTrpAspAsnArgThrIleIleAsnProIleSerGlyLeuVal
     5-AlaThrArgTrpGlnIleTrpAspAsnGlyThrIleIleAsnProArgSerGlyLeuVal

       TTGGCAGCCACATCAGGAAACAGTGGTACCACACTTACAGTGCAAACCAACATTTATGCC
     4-LeuAlaAlaThrSerGlyAsnSerGlyThrThrLeuThrValGlnThrAsnIleTyrAla
     5-LeuAlaAlaThrSerGlyAsnSerGlyThrThrLeuThrValGlnThrAsnIleTyrAla

       GTTAGTCAAGGTTGGCTTCCTAGTAATAATACACAACCTTTTGTGACATCCATTGTTGGG
     4-ValSerGlnGlyTrpLeuProSerAsnAsnThrGlnProPheValThrSerIleValGly
     5-ValSerGlnGlyTrpLeuProThrAsnAsnThrGlnProPheValThrThrIleValGly

       CTAAATGATCTCTGTTTACAAGCAAATACTGGAAAAGTATGGTTAGACGAGTGTACAAGT
     4-LeuAsnAspLeuCysLeuGlnAlaAsnThrGlyLysValTrpLeuAspGluCysThrSer
     5-LeuTyrGlyLeuCysLeuGlnAlaAsnSerGlyGlnValTrpIleGluAspCysSerSer

       GAAAAGGCTGAACAACAATGGGCGCTTTATGCAGATGGTTCAATACGGCCTCAGCAAAAC
     4-GluLysAlaGluGlnGlnTrpAlaLeuTyrAlaAspGlySerIleArgProGlnGlnAsn
     5-GluLysAlaGluGlnGlnTrpAlaLeuTyrAlaAspGlySerIleArgProGlnGlnAsn

       CAAGATAACTGCCTTACAAGTGATGCTAATATACGAGAAACAATTGTCAAGACCCTCTCT
     4-GlnAspAsnCysLeuThrSerAspAlaAsnIleArgGluThrIleValLysThrLeuSer
     5-ArgAspAsnCysLeuThrSerAspSerAsnIleArgGluThrValValLysIleLeuSer

       TGCAGCACTGCATCCTCCGGCCAGCGATGGATGTTCAAGAATGATGGAACCATTTGGAAT
     4-CysSerThrAlaSerSerGlyGlnArgTrpMetPheLysAsnAspGlyThrIleTrpAsn
     5-CysGlyProAlaSerSerGlyGlnArgTrpMetPheLysAsnAspGlyThrIleLeuAsn

       TTGTATAATGGATTGGTGTTAGATGTGAAGCGATCGGATCCGACCCTTAAACAAATCATT
     4-LeuTyrAsnGlyLeuValLeuAspValLysArgSerAspProThrLeuLysGlnIleIle
     5-LeuTyrSerGlyLeuValLeuAspValArgAlaSerAspProSerLeuLysGlnIleIle

       ATTTACCCTTTCCATGGAAACCCAAACCAAATATGGTTTCCACTATTTTGATAGACTAAT
     4-IleTyrProPheHisGlyAsnProAsnGlnIleTrpPheProLeuPhe
     5-LeuTyrProLeuHisGlyAspProAsnGlnIleTrpLeuProLeuPhe

       TACCCTCTTGCAGTGTATGTATGTCCTACCATGAACATAGTTGCTTAAATAAAAAGGACA

       TTGTAAATTAAAAAAAAAAAAAAAAA
```

FIG. 5 Junction Region for the Construction of pRTB601

```
                                                        |-----   pRTB-151   --->
       Hind III  |--------------   ricin-B   ---------------------------->
                    MetAlaAspValCysMetAspProGluProIleValArgIleValGlyArgAsnGlyLeu...
GACCATGATAAGCTTATGGCTGATGTTTGTATGGATCC          GCGTATCGTAGGTCGAAATGGTCTA...
                                 TACCTAGGACTCGGGTATCACGCATAGCATCC       ACCAGAT...
       (oligo 2)                           (oligo 1)
```

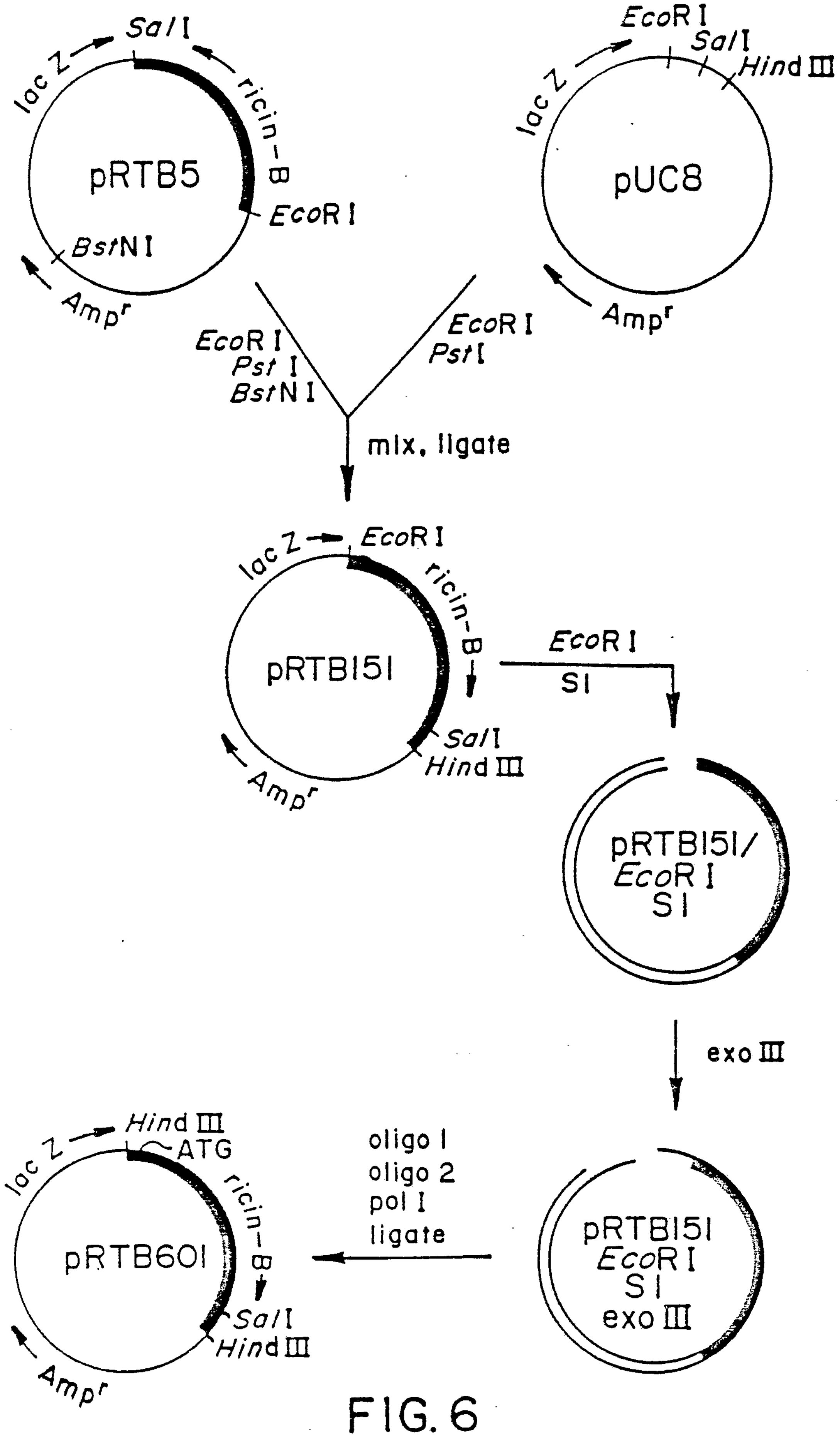

Sal I
lac Z
ricin-B
pRTB5
EcoR I
BstN I
Amp^r
EcoR I
Sal I
Hind III
lac Z
pUC8
Amp^r
EcoR I
Pst I
BstN I
EcoR I
Pst I
mix, ligate
lac Z
EcoR I
ricin-B
pRTB151
Sal I
Hind III
Amp^r
EcoR I
S1
pRTB151/
EcoR I
S1
exo III
pRTB151
EcoR I
S1
exo III
oligo 1
oligo 2
pol I
ligate
Hind III
ATG
lac Z
ricin-B
pRTB601
Sal I
Hind III
Amp^r


FIG. 6